# EUROPEAN PATENT SPECIFICATION

(11) **EP 3 701 967 B1**
(45) Date of publication and mention of the grant of the patent: **30.07.2025**
(21) Application number: 19159260.9
(22) Date of filing: 26.02.2019
(51) Int. Cl.: A61K 38/16

(54) **USE OF JACK BEAN LECTIN FOR INCREASING THE ABUNDANCE OF HEMATOPOIETIC STEM CELLS AND PROGENITOR CELLS IN BONE MARROW AND/OR EPIDERMAL STEM CELLS IN SKIN IN VIVO**
VERWENDUNG VON SCHWERTBOHNENLECTIN ZUR ERHÖHUNG DER MENGE HÄMATOPOIETISCHER STAMMZELLEN UND VORLÄUFERZELLEN IN KNOCHENMARK UND/ODER EPIDERMALER STAMMZELLEN IN DER HAUT IN VIVO
UTILISATION DE LECTINE EXTRAITE DE FÈVES VELOUTÉES POUR AUGMENTER L'ABONDANCE DE CELLULES SOUCHES HÉMATOPOÏÉTIQUES ET DE CELLULES PROGÉNITRICES DANS LA MOELLE OSSEUSE ET/OU DE CELLULES SOUCHES ÉPIDERMIQUES DANS LA PEAU IN VIVO

(43) Date of publication of application: 02.09.2020
(73) Proprietor: THE SECRETARY, DEPARTMENT OF ATOMIC ENERGY, Mumbai 400 001 (IN)
(72) Inventor: Sharma, Deepak, 400085 Mumbai (IN); Sandur, Santosh Kumar, 400085 Mumbai (IN); Thoh, Maikho, 400085 Mumbai (IN); Patwardhan, Raghavendra Shridhar, 400085 Mumbai (IN); Maurya, Dharmendra K., 400085 Mumbai (IN); Checker, Rahul, 400085 Mumbai (IN); Gota, Vikram P., 410210 Navi Mumbai (IN); Sundarraj, Jayakumar, 400085 Mumbai (IN); Sarma, Haldhar Dev, 400085 Mumbai (IN); Chattopadhyay, Subrata, 400085 Mumbai (IN)
(74) Representative: Petraz, Gilberto Luigi

(56) References cited:
- US-A- 4 889 842
- LUZ MARINA MELGAREJO ET AL: "Isolation and characterization of novel lectins from Canavalia ensiformis DC and Dioclea grandiflora Mart. ex Benth. seeds", BRAZILIAN JOURNAL OF PLANT PHYSIOLOGY, vol. 17, no. 3, 1 January 2005 (2005-01-01), pages 315 - 324, XP055606952, DOI: 10.1590/S1677-04202005000300006
- TOSHIKI SAKAMOTO ET AL: "Concanavalin A simultaneously primes liver hematopoietic and epithelial progenitor cells for parallel expansion during liver regeneration after partial hepatectomy in mice", HEPATOLOGY, vol. 32, no. 2, 1 August 2000 (2000-08-01), pages 256 - 267, XP055606957, ISSN: 0270-9139, DOI: 10.1053/jhep.2000.9406
- YOH-ICHI TAGAWA ET AL: "Bimodal role of endogenous interleukin-6 in concanavalin A-induced hepatitis in mice", JOURNAL OF LEUKOCYTE BIOLOGY, vol. 67, no. 1, 1 January 2000 (2000-01-01), US, pages 90 - 96, XP055606965, ISSN: 0741-5400, DOI: 10.1002/jlb.67.1.90
- YANZHEN BI ET AL: "Human liver stem cells attenuate concanavalin A-induced acute liver injury by modulating myeloid-derived suppressor cells and CD4+ T cells in mice", STEM CELL RESEARCH & THERAPY, vol. 10, no. 1, 11 January 2019 (2019-01-11), XP055606963, DOI: 10.1186/s13287-018-1128-2
- K. SEKIYA ET AL: "Enhancement of Osteogenesis by Concanavalin a in Human Bone Marrow Mesenchymal Stem Cell Cultures", INTERNATIONAL JOURNAL OF ARTIFICIAL ORGANS, vol. 31, no. 8, 10 August 2008 (2008-08-10), IT, pages 708 - 715, XP055606807, ISSN: 0391-3988, DOI: 10.1177/039139880803100804

## Description

### FIELD OF INVENTION

The invention relates to a method for increasing the abundance of hematopoietic stem cells and progenitor cells in the bone marrow and skin stem cells. More specifically the present invention provides the use of Jack bean lectin or a pharmaceutically acceptable salt thereof as an injectable formulation for use in the treatment of bone marrow aplasia and deficiency of skin stem cells.

### BACKGROUND & PRIOR ART

The use of stem cells in regenerative medicine for the treatment of various human diseases is well established. The aim of regenerative medicine is to restore normal tissue functions by replenishing injured tissues with healthy cells using either cell-based therapy or by inducing endogenous repair and regeneration using small molecules. The approach of inducing endogenous repair and regeneration requires in vivo modulation of tissue-specific stem cells by therapeutic agents and enhance their abundance through activation, proliferation, differentiation, or reprogramming. Several candidate molecules like 2 deoxy-D-glucose, quercetin, baicalein, chlorophyllin, fructose 2,6-bisphosphate, rapamycin and 5-aminoimidazole-4-carboxamide ribonucleotide have been shown to enhance reprogramming and pluripotent stem cell induction (Liu K et al., Cell Chem Biol. 2016 Aug 18;23(8):893-916). Small molecule activators of lineage specific differentiation and lineage conversion are available for neural, cardiac, myogenic, pancreatic or hepatic tissues (Wagner JE Jr et al., Cell Stem Cell. 2016 Jan 7;18(1):144-55). These candidate molecules have been shown to allow ex vivo expansion of stem cells. A synthetic purine derivative, StemRegenin 1 promotes in vitro expansion of CD34+ hematopoietic stem cells in combination with cytokines involved in hematopoiesis by antagonizing aryl hydrocarbon receptor signaling (Boitano AE et al., Science. 2010 Sep 10;329(5997):1345-8). UM171, a pyrimidoindole derivative can expand HSC independent of AhR signaling in vitro (Fares I et al., Science. 2014 Sep 19;345(6203):1509-12). CD4+ T helper cells have been shown to modulate hematopoiesis by improving the HSC transplantation and increasing granulopoiesis through secreted cytokines including oncostatinM (OSM) (Broxmeyer HE et al., Immunity. 2002 Jun;16(6):815-25). On the contrary, Treg cells inhibit differentiation and preserve HSC pool in the bone marrow (Bonomo et al., 2016 Frontiers in Immunology, 7: 184).

Experimental as well as translational approaches are available for modulation of intrinsic stem cell numbers, differentiation, lineage conversion, pluripotency induction and directed differentiation. These approaches include overexpression of specific transcription factors (Oct4, Nanog, Sox2, Klf4 and c-Myc, OSKM), epigenetic modifications (using 5-Azacytidine and RG108), blocking TGF-β receptor signaling, inhibition of GSK3, activation of Wnt and activation of MAPK/ERK pathway using thiazovivin (Zhang Y et al., J Cell Sci 2012 125: 5609-5620). The galactoside-specific plant lectin, Viscum album agglutinin (VAA-I) has been shown to increase the clonogenic proliferation of hematopoietic stem cells in combination with growth factors and cytokines. However, VAA-1 alone did not increase HSC proliferation (Vehmeyer K et al., Eur J Haematol. 1998 Jan;60(1):16-20). Currently Granulocyte colony stimulation factor (GCSF)is used for increasing the number of hematopoietic stem cells in circulation before harvesting CD34+ cells for adoptive transfer (Brissot E et al., Bone Marrow Transplant. 2009 Nov;44(9):613-5.). However, formulations are not available for increasing the abundance of intrinsic hematopoietic stem cells in the bone marrow.

In spite of various methods known in the art to dedifferentiate, transdifferentiate or re-differentiate the cells, there are very limited options for increasing the abundance of hematopoietic stem cells in the bone marrow. Formulations are not available for increasing the abundance of epidermal stem cells in the skin.

Lectins are carbohydrate-binding proteins, and their interaction with any cell is dependent on the particular types of carbohydrate moieties present on the cell surface. Blasco E et al., demonstrates that Jacalin lectin bind to β-galactoside on the surface of T-lymphocytes and it leads to proliferation of T-lymphocytes (Blasco E et al., Eur J Immunol. 1995 Jul;25(7):2010-8). Sanford et al states that the lectins from peanut and mushroom, binds to β-galactoside present in the vascular cell and lead to their proliferation (Sanford GL., FASEB J. 1990 Aug;4(11):2912-8). These arts further teach that the interactions between lectin and cell is both cell type specific and lectin specific. Our data shows that Jack bean lectin can bind to similar percentage of T cells, B cells and macrophages. However, it is known to induce proliferation only in T cells and not in B cells and macrophages. This data teaches that mere binding of lectin to a specific cell type is not sufficient to induce proliferation in the mammalian cells. Many lectins are known to bind to different types of tissue stem cells but they did not induce any proliferation. Eg: Peanut lectin binds mammary epithelial stem cells in rats (Sharma et al., PLoS One. 2011;6(10):e26145; Smits BM et al., PLoS Genet. 2013 Jun;9(6):e1003549; Wang YC et al., Cell Res. 2011 Nov;21(11):1551-63).

Lectins are known to bind to glycoproteins on the surface of a variety of cells including bacteria, mammalian lymphocytes and intestinal brush border cells (Pusztai et al., Plant Lectins, 1991, Cambridge University Press, Cambridge, UK.). Lectins like phytohemagglutinin from Kidney bean can cause agglutination of erythrocytes and T lymphocytes. Lectins from Jack bean and red kidney bean are known to induce proliferation in T cells (Greaves MF et al., Nature (Lond.), 248, 698-701; and Palacios R, J Immunol. 1982 Jan;128(1):337-42). Peanut lectin is known to bind to mammary epithelial cells including stem cells (Kim M et al., Glycobiology. 1993 Oct;3(5):447-53; Sharma et al., PLoS One. 2011;6(10):e26145). A lectin from Kidney beans has also been shown to induce mucosal cell proliferation in the reduction and/or treatment of mucositis and/or gut lesions (CA2258503C). However, the effect of lectin from Kidney bean on stem cells has not been mentioned or claimed (CA2258503C).

The Lectin isolated from jack-bean *Canavalia ensiformis* specifically binds to various sugars, glycoproteins and glycolipids, mainly internal and non-reducing terminal α-D-mannosyl and α-D-glucosyl groups. It is widely used to characterize glycoproteins and other sugar containing entities on the surface of various cells.

Melgarejo LM et al., Braz. J. Plant Physiol. 2005; 17(3): 315-324). discloses isolation of, two lectins from seeds of phylogenetically close plants *Canavalia ensiformis* and *Diocleinae grandiflora* having a functional similarity.

The said lectin is a homotetramer made from four sub-units each consisting of 235 amino acids (26.5KDa). It is highly glycosylated and binds a divalent metal ion (Mn²⁺ or Ca²⁺). It can bind to glycoproteins on the surface of lymphocytes and initiate mitogenesis principally acting on T-lymphocytes by stimulating energy metabolism. The lectin interacts with different mannose containing receptors like rhodopsin, blood group markers, insulin receptor, immunoglobulins, carcinoembryonic antigen (CEA), lipoproteins and T cell receptor. It can agglutinate erythrocytes in a dose and energy dependent manner.

The said lectin induces T cell receptor cross linking, membrane reorganization and NF-kB mediated signaling leading to activation, cytokine production and cell cycle progression in T cells. It can bind to many microbes including *E. coli, B. subtilis* and also protists like *D. discoideum.*

Sakamoto T et al., (Hepatology. 2000 Aug;32(2):256-67) discloses that Con A pretreatment primed liver hematopoietic progenitor cells and liver epithelial cells for a rapid and parallel expansion.

It has been demonstrated to exhibit therapeutic effect against experimental hepatoma. It was found to sequester more hepatic tumor cells in preference to normal hepatocytes. It is identified as reproductive hazard 3, known to cause allergy, asthma or breathing difficulties, if inhaled. It may also cause toxicity to unborn child in pregnant females. It can cause skin irritation, eye irritation or irritation in the mucous membranes and upper respiratory tract. The LD50 intraperitoneal dose (rat) is 41.5mg/kg, intravenous LD50 (mouse) is 50 mg/kg, intravenous lowest published toxic dose (TDLO) in female rats 8 days after conception is 10mg/kg and in female mice 7 days after conception is 5mg/kg in the literature. Lectin induces unscheduled DNA synthesis in lymphocytes.

Toxic effects of the lectin have been studied earlier in detail. At higher doses, it can cause damage to liver primarily due to activation and infiltration of T cells, production of TNF-α and IL-6 leading to sinusoidal occlusion and increased transaminase release. P selectin has been shown to play a causal role in mediating lectin mediated hepatitis. However, there is no disclosure on the effect of said lectin on increasing the abundance of hematopoietic cells of bone marrow and epidermal stem cells of skin.

There is still a need for developing effective methods of increasing the abundance of hematopoietic and skin stem cells using specific lectins from Jack bean for treating various health conditions due to depletion of said stem cells.

### OBJECT OF INVENTION

It is thus the primary object of the present invention to provide use of Jack bean lectin or a pharmaceutically acceptable salt thereof for increasing the abundance of hematopoietic stem cells in bone marrow and/or epidermal stem cells in skin in vivo.

Another object of the present invention is to provide a method of treatment of disease condition resulting from deficiency of hematopoietic stem and progenitor cells and/or epidermal stem cells in skin.

A further object of the present invention is to provide the method of treatment for bone marrow aplasia and deficiency of skin stem cells.

Yet another object of the present invention is to provide the method of treatment of said disease conditions comprising administering a single injection of lectin dose to increase the abundance of skin stem cells.

A further object of the present invention is to provide for manufacture of injectable formulation comprising of the Jack bean lectin or a pharmaceutically acceptable saltthereof.

### SUMMARY OF THE INVENTION

Thus, according to the basic aspect of the present invention there is provided an injectable formulation comprising the Jack Bean derived lectin of Seq. ID. No. 1:
Ala Asp Thr Ile Val Ala Val Glu Leu Asp Thr Tyr Pro Asn Thr Asp Ile Gly Asp Pro Ser Tyr Pro His Ile Gly Ile Asp Ile Lys Ser Val Arg Ser Lys Lys Thr Ala Lys Trp Asn Met Gln Asn Gly Lys Val Gly Thr Ala His Ile Ile Tyr Asn Ser Val Asp Lys Arg Leu Ser Ala Val Val Ser Tyr Pro Asn Ala Asp Ser Ala Thr Val Ser Tyr Asp Val Asp Leu Asp Asn Val Leu Pro Glu Trp Val Arg Val Gly Leu Ser Ala Ser Thr Gly Leu Tyr Lys Glu Thr Asn Thr Ile Leu Ser Trp Ser Phe Thr Ser Lys Leu Lys Ser Asn Thr His Glu Thr Asn Ala Leu His Phe Met Asn Gln Phe Ser Lys Asp Gln Lys Asp Leu Ile Leu Gln Gly Asp Ala Thr Thr Gly Thr Asp Gly Asn Leu Glu Leu Thr Arg Val Ser Ser Asn Gly Ser Pro Gln Gly Ser Ser Val Gly Arg Ala Leu Phe Tyr Ala Pro Val His Ile Trp Glu Ser Ser Ala Val Val Ala Ser Phe Glu Ala Thr Phe Thr Phe Leu Ile Lys Ser Pro Asp Ser His Pro Ala Asp Gly Ile Ala Phe Phe Ile Ser Asn Ile Asp Ser Ser Ile Pro Ser Gly Ser Thr Gly Arg Leu Leu Gly Leu Phe Pro Asp Ala Asn or a pharmaceutically acceptable salt thereof in amounts of 1 to 10, 10 to 100, 50 to 250 mg for use in the treatment of disease of bone marrow aplasia and deficiency of skin stem cells.

### DETAILED DESCRIPTION OF THE INVENTION

As discussed hereinbefore the present advancement provides for the first time the novel use of Jack bean lectin or a pharmaceutically acceptable salt thereof for increasing the abundance of hematopoietic stem cells and progenitor cells in bone marrow and/or epidermal stem cells in skin in vivo and a method of treatment involving the said lectin at a specific dose to treat conditions due to depletion of stem cells.

The said lectin derived from Jack bean or a pharmaceutically acceptable salt thereof has the sequence given below (Seq.. No. Id 1).

Ala Asp Thr Ile Val Ala Val Glu Leu Asp Thr Tyr Pro Asn Thr Asp Ile Gly Asp Pro
Ser Tyr Pro His Ile Gly Ile Asp Ile Lys Ser Val Arg Ser Lys Lys Thr Ala Lys Trp
Asn Met Gln Asn Gly Lys Val Gly Thr Ala His Ile Ile Tyr Asn Ser Val Asp Lys Arg
Leu Ser Ala Val Val Ser Tyr Pro Asn Ala Asp Ser Ala Thr Val Ser Tyr Asp Val Asp
Leu Asp Asn Val Leu Pro Glu Trp Val Arg Val Gly Leu Ser Ala Ser Thr Gly Leu
Tyr Lys Glu Thr Asn Thr Ile Leu Ser Trp Ser Phe Thr Ser Lys Leu Lys Ser Asn
Thr His Glu Thr Asn Ala Leu His Phe Met Asn Gln Phe Ser Lys Asp Gln Lys Asp
Leu Ile Leu Gln Gly Asp Ala Thr Thr Gly Thr Asp Gly Asn Leu Glu Leu Thr Arg
Val Ser Ser Asn Gly Ser Pro Gln Gly Ser Ser Val Gly Arg Ala Leu Phe Tyr Ala
Pro Val His Ile Trp Glu Ser Ser Ala Val Val Ala Ser Phe Glu Ala Thr Phe Thr Phe
Leu Ile Lys Ser Pro Asp Ser His Pro Ala Asp Gly Ile Ala Phe Phe Ile Ser Asn Ile
Asp Ser Ser Ile Pro Ser Gly Ser Thr Gly Arg Leu Leu Gly Leu Phe Pro Asp Ala
Asn

As used herein, the term "hematopoietic stem cells and progenitor cells" or "HSPC's" refers to the cells in the bone marrow giving rise to all the types of blood cells, including red blood cells, B lymphocytes, T lymphocytes, natural killer cells, neutrophils, basophils, eosinophils, monocytes, and macrophages.

As used herein, the term "skin stem cells" or "SSC" refers to the cells in the skin giving rise to different layers of epidermis. These stem cells are found in the basal layer of epidermis.

As used herein, the term "increase" refers to increase in the number of cells or units, concentration, activity, intensity, frequency, population, or cellularity, whichever is applicable, by at least 10%, 20 %, 30 %, 40 %, 50 %, 60 %, 70 % or 80 % as compared to that measured before or at the time of administration of pharmaceutical composition of the invention.

As used herein, the term "abundance of cells or units" refers to the number of cells or units in the bone marrow or skin at a given time.

The term "animal" is used herein to include all vertebrate mammals, including human.

The said lectin or a pharmaceutically acceptable salt thereof may be administered alone or in combination with pharmaceutically acceptable carriers, vehicles or diluents, in either single or multiple doses. Suitable pharmaceutical carriers, vehicles and diluents include inert solid diluents or fillers, sterile aqueous solutions and various organic solvents. The pharmaceutical compositions formed by combining the compositions and the pharmaceutically acceptable carriers, vehicles or diluents are then readily administered in a variety of dosage forms such as tablets, powders, lozenges, syrups, injectable solutions.

For parenteral administration, solutions of the compositions may be prepared in (for example) aqueous propylene glycol, or sterile aqueous solutions may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration, in this connection; the sterile aqueous media employed are all readily available and are known to those skilled in the art.

The compositions, for instance injectable, may contain e.g. 1 to 10, 10 to 100, 50 to 250 mg of the lectin or pharmaceutical acceptable salts of the constituent peptide sequence thereof.

Generally, a composition as described herein may be administered parenterally (e.g., intravenous, intramuscular, subcutaneous or intramedullary). The most preferred route of administration is intravenous.

The details of the invention are described as hereunder in the accompanying figures and illustrative examples.

### BRIEF DESCRIPTION OF ACCOMPANYING FIGURES

Fig. 1A represents percent hematopoietic stem cells in the bone marrow compartment of mice 24hrs after injectionof different doses of lectin.
Fig 1B represents effect of lectin on hematopoietic stem cells in the bone marrow compartment of mice injected with8mg/kg dose at different time points.
Figure 2A illustrates effect of different doses of lectin on abundance of skin stem cells.
Figure 2B.: Changes in the abundance of skin stem cells in the skin in response to 0.5 mg/kg,2 mg/kg or 4 mg/kg body weight dose of lectin at different time points.
Figure 3 A and 3B illustratethat the lectin binds to more than 96 % of the CD3+ T cells, CD19+ B cells as well as CD14+ macrophages.

### Animal maintenance:

Eight- to 9-weeks-old inbred Swiss albino mice weighing approximately 20-25 g, reared in the animal house of Bhabha Atomic Research Centre were used. Mice were housed at constant Temperature (23°C) with a 12h / 12h light /dark cycle and were given mouse chow and water ad libitum.

### Statistical Analysis:

Data are presented as mean ± SEM. Statistical analysis was done using ANOVA for comparing multiple treatment groups using GraphPad Prism 6 software.

In preparing the injectable formulation, phosphate buffered saline pH 7.2 has been used to dissolve the lyophilized lectin powder to facilitate the delivery of lectin. The solvent can be replaced by sterile isotonic saline solution.

### EXAMPLE 1: Assessment of hematopoietic stem cell abundance:

Mice injected with lectin dose of 0.5, 1.0, 2.0, 4.0, 8.0 or 10.0 mg / kg body weight were sacrificed at indicated time points, femur bones were excised, bone marrow mononuclear cells (BM-MNC) were flushed out and counted by trypan blue viable counting method. SLAM (signaling lymphocyte activation marker) staining of BM-MNC was performed using monoclonal antibodies against lineage markers, CD48, CD244 & CD150 conjugated with different fluorochromes. Briefly, 2.5 million cells were resuspended in IMDM with 10% FBS for blocking on ice for 10 min. Cells were spin down and resuspended in antibody staining solution (20µl per tube/0.2µg per sample) and incubated on ice for 30 min. Cells were washed and resuspended in 1X PBS, fixed with 4% formaldehyde, washed again with PBS and Hoechst 33342 dye (10µg/ml) was added for nuclear staining. Single stained, unstained and fluorescence minus one (FMO) control were kept for compensation during flow cytometric analysis.

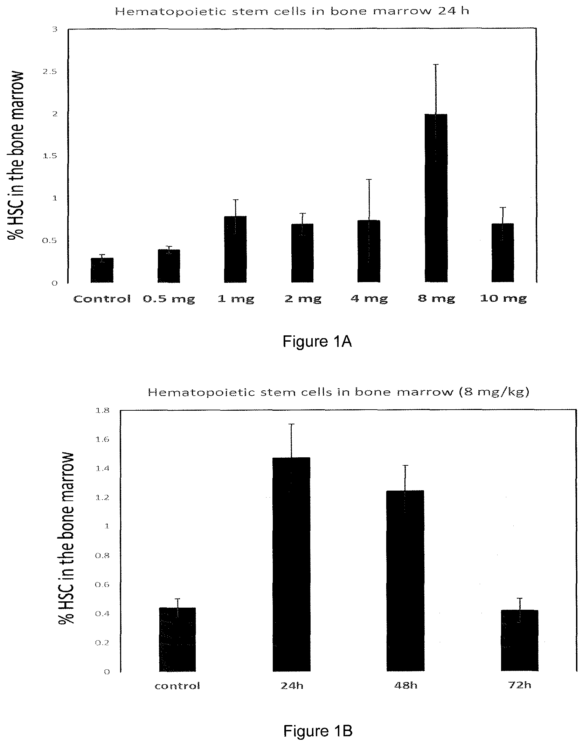

Fig. 1A shows the effect of lectin at different doses on the percentage of hematopoietic stem and progenitor cells in the bone marrow of miceat 24 hrs. Maximum increase is seen at the dose of 8mg/Kg body wtillustrated in Fig. 1B. Injection of lectin formulation to mice significantly enhanced the abundance of Lineage⁻CD244⁻CD48⁻CD150⁺ hematopoietic stem cells at 24h. This increase in the frequency of bone marrow HSCs is transient since their percentage restored back to normal levels at 72h. **(****FIGURE 1A and 1B****)**

### EXAMPLE 2: Assessment of skin stem cell abundance:

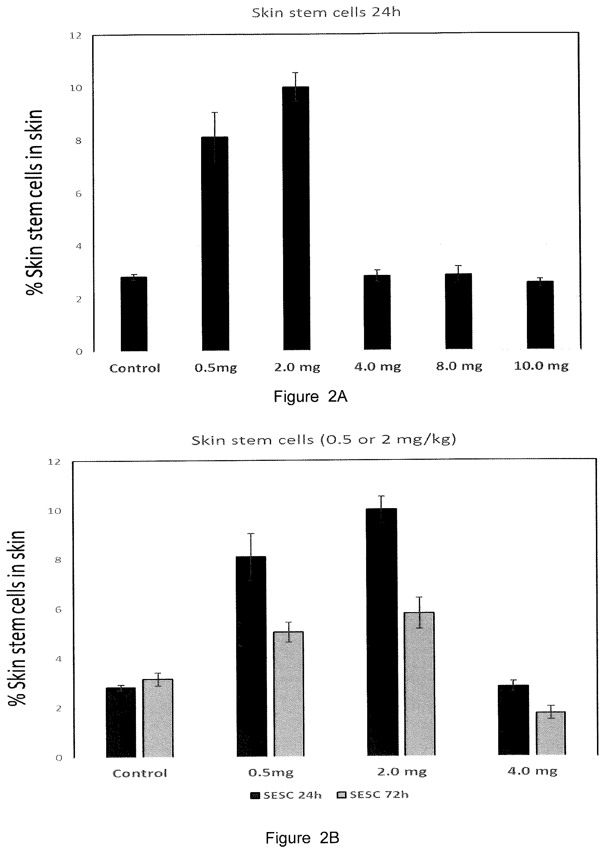

Mice injected with different doses of lectin were sacrificed, hair was removed, skin was excised from back side and kept for overnight incubation in 1X trypsin-EDTA solution at 4°C. Plate containing skin and trypsin solution was shifted to 37⁰C and incubated for 1h. After 1h, complete DMEM was added to neutralize trypsin and skin cells were removed by scraping. Cells were spun down in centrifuge at 2500 rpm/5 min/RT and counted by trypan blue viable counting method. Cells were stained with fluorochrome conjugated monoclonal antibodies against CD34 and CD49f followed by fixation and Hoechst 33342 nuclear staining as described in the above protocol. Single stained and unstained controls were kept for compensation in flow cytometric data analysis. Fig. 2 shows the ability of the said lectin to enhance abundance of intrinsic skin stem cells. Figure 2A illustrates effect of different doses of lectin on abundance of skin stem cells. Figure 2B.: Changes in the abundance of skin stem cells in the skin in response to 0.5 mg/kg or 2 mg/kg body weight dose of lectin at different time points

Mice administered with two different doses of lectin formulation showed increase in the percentage of CD34+CD49f+ skin epithelial stem cells in the skin. **(****FIGURE 2A and 2B****).**

A single dose of lectin was sufficient to achieve the increase in abundance of skin stem cells and bone marrow hematopoietic stem cells. The present findings are peculiar and unexpected because the dose required to increase the abundance of hematopoietic stem cells is almost double the dose required to enhance the abundance of skin stem cells.

### EXAMPLE 3: Assessment of lectin binding to cells:

Lectin (100ug) was conjugated with Dylight488 using the kit (ThermoFisher Cat No 53024). Splenic lymphocytes from mice were stained with lectin (1ug) and fluorochrome conjugated monoclonal antibodies against CD3 (PE-cy5) or CD19 (PE) or CD14 (PE) followed by fixation and Hoechst 33342 nuclear staining as described in the above protocol. Single stained and unstained controls were kept for compensation in flow cytometric data analysis.

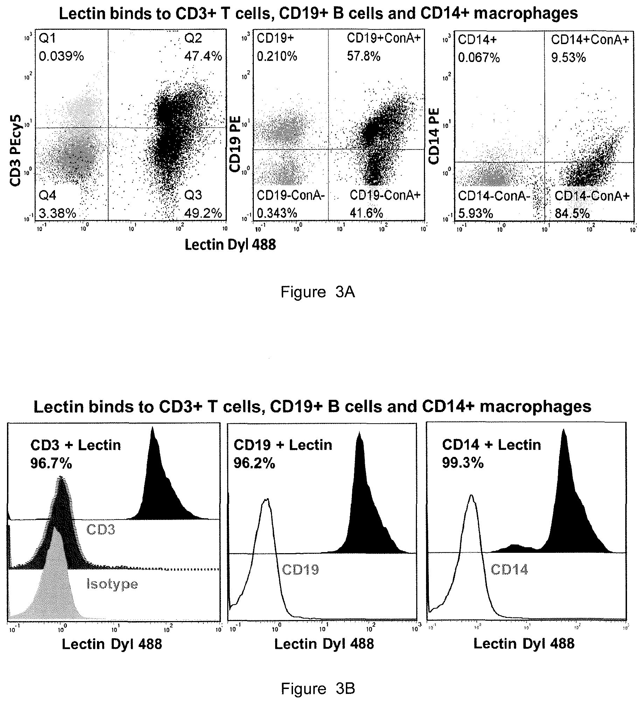

Fig. 3A shows the two parameter dot plots depicting binding of lectin to CD3+ T cells, CD19+ B cells and CD14+ macrophages. Figure 2B illustrates overlaid flow cytometric histograms showing binding of lectin to more than 96% T cells, B cells and macrophages.

It is thus possible for the present advancement to provide for a method of treatment administering select dose of Jack bean lectin for treating disease condition resulting from deficiency of hematopoietic stem and progenitor cells in the bone marrow and/or epidermal stem cells in skin in vivo for the first time.

## Claims

1. An injectable formulation comprising the Jack Bean derived lectin of Seq. ID. No. 1:
Ala Asp Thr Ile Val Ala Val Glu Leu Asp Thr Tyr Pro Asn Thr Asp Ile Gly Asp Pro Ser Tyr Pro His Ile Gly Ile Asp Ile Lys Ser Val Arg Ser Lys Lys Thr Ala Lys Trp Asn Met Gln Asn Gly Lys Val Gly Thr Ala His Ile Ile Tyr Asn Ser Val Asp Lys Arg Leu Ser Ala Val Val Ser Tyr Pro Asn Ala Asp Ser Ala Thr Val Ser Tyr Asp Val Asp Leu Asp Asn Val Leu Pro Glu Trp Val Arg Val Gly Leu Ser Ala Ser Thr Gly Leu Tyr Lys Glu Thr Asn Thr Ile Leu Ser Trp Ser Phe Thr Ser Lys Leu Lys Ser Asn Thr His Glu Thr Asn Ala Leu His Phe Met Asn Gln Phe Ser Lys Asp Gln Lys Asp Leu Ile Leu Gln Gly Asp Ala Thr Thr Gly Thr Asp Gly Asn Leu Glu Leu Thr Arg Val Ser Ser Asn Gly Ser Pro Gln Gly Ser Ser Val Gly Arg Ala Leu Phe Tyr Ala Pro Val His Ile Trp Glu Ser Ser Ala Val Val Ala Ser Phe Glu Ala Thr Phe Thr Phe Leu Ile Lys Ser Pro Asp Ser His Pro Ala Asp Gly Ile Ala Phe Phe Ile Ser Asn Ile Asp Ser Ser Ile Pro Ser Gly Ser Thr Gly Arg Leu Leu Gly Leu Phe Pro Asp Ala Asn or a pharmaceutically acceptable salt thereof in amounts of 1 to 10, 10 to 100, 50 to 250 mg for use in the treatment of disease of bone marrow aplasia and deficiency of skin stem cells.

## Patentansprüche

1. Injizierbare Formulierung umfassend das von Schwertbohne abgeleitete Lektin der Seq. ID. Nr. 1:
Ala Asp Thr Ile Val Ala Val Glu Leu Asp Thr Tyr Pro Asn Thr Asp Ile Gly Asp Pro Ser Tyr Pro His Ile Gly Ile Asp Ile Lys Ser Val Arg Ser Lys Lys Thr Ala Lys Trp Asn Met Gln Asn Gly Lys Val Gly Thr Ala His Ile Ile Tyr Asn Ser Val Asp Lys Arg Leu Ser Ala Val Val Ser Tyr Pro Asn Ala Asp Ser Ala Thr Val Ser Tyr Asp Val Asp Leu Asp Asn Val Leu Pro Glu Trp Val Arg Val Gly Leu Ser Ala Ser Thr Gly Leu Tyr Lys Glu Thr Asn Thr Ile Leu Ser Trp Ser Phe Thr Ser Lys Leu Lys Ser Asn Thr His Glu Thr Asn Ala Leu His Phe Met Asn Gln Phe Ser Lys Asp Gln Lys Asp Leu Ile Leu Gln Gly Asp Ala Thr Thr Gly Thr Asp Gly Asn Leu Glu Leu Thr Arg Val Ser Ser Asn Gly Ser Pro Gln Gly Ser Ser Val Gly Arg Ala Leu Phe Tyr Ala Pro Val His Ile Trp Glu Ser Ser Ala Val Val Ala Ser Phe Glu Ala Thr Phe Thr Phe Leu Ile Lys Ser Pro Asp Ser His Pro Ala Asp Gly Ile Ala Phe Phe Ile Ser Asn Ile Asp Ser Ser Ile Pro Ser Gly Ser Thr Gly Arg Leu Leu Gly Leu Phe Pro Asp Ala Asn oder ein pharmazeutisch akzeptables Salz davon in Mengen von 1 bis 10, 10 bis 100, 50 bis 250 mg zur Verwendung bei der Behandlung der Krankheit der Knochenmarkaplasie und des Mangels an Hautstammzellen.

## Revendications

1. Formulation injectable comprenant la lectine issue du pois-sabre de Seq. ID. No. 1 :
Ala Asp Thr Ile Val Ala Val Glu Leu Asp Thr Tyr Pro Asn Thr Asp Ile Gly Asp Pro Ser Tyr Pro His Ile Gly Ile Asp Ile Lys Ser Val Arg Ser Lys Lys Thr Ala Lys Trp Asn Met Gln Asn Gly Lys Val Gly Thr Ala His Ile Ile Tyr Asn Ser Val Asp Lys Arg Leu Ser Ala Val Val Ser Tyr Pro Asn Ala Asp Ser Ala Thr Val Ser Tyr Asp Val Asp Leu Asp Asn Val Leu Pro Glu Trp Val Arg Val Gly Leu Ser Ala Ser Thr Gly Leu Tyr Lys Glu Thr Asn Thr Ile Leu Ser Trp Ser Phe Thr Ser Lys Leu Lys Ser Asn Thr His Glu Thr Asn Ala Leu His Phe Met Asn Gln Phe Ser Lys Asp Gln Lys Asp Leu Ile Leu Gln Gly Asp Ala Thr Thr Gly Thr Asp Gly Asn Leu Glu Leu Thr Arg Val Ser Ser Asn Gly Ser Pro Gln Gly Ser Ser Val Gly Arg Ala Leu Phe Tyr Ala Pro Val His Ile Trp Glu Ser Ser Ala Val Val Ala Ser Phe Glu Ala Thr Phe Thr Phe Leu Ile Lys Ser Pro Asp Ser His Pro Ala Asp Gly Ile Ala Phe Phe Ile Ser Asn Ile Asp Ser Ser Ile Pro Ser Gly Ser Thr Gly Arg Leu Leu Gly Leu Phe Pro Asp Ala Asn ou sel pharmaceutiquement acceptable de celle-ci en quantités de 1 à 10, 10 à 100, 50 à 250 mg pour utilisation dans le traitement d'une maladie du type aplasie médullaire et de la déficience en cellules souches de la peau.
